# EUROPEAN PATENT SPECIFICATION

(11) **EP 2 838 507 B1**
(45) Date of publication and mention of the grant of the patent: **30.05.2018**
(21) Application number: 13727984.0
(22) Date of filing: 17.04.2013
(51) Int. Cl.: A61K 31/70, A61K 9/00, A61K 9/107, A61K 9/70

(54) **MATRIX AND DEVICE AND USE THEREOF FOR OPTICALLY-CONTROLLED RELEASE OF CHEMICALS**
MATRIX UND VORRICHTUNG SOWIE VERWENDUNG ZUR OPTISCH GESTEUERTEN FREISETZUNG VON CHEMISCHEN STOFFEN
MATRICE ET DISPOSITIF, ET LEUR UTILISATION POUR LA LIBÉRATION OPTIQUEMENT CONTRÔLÉE DE PRODUITS CHIMIQUES

(30) Priority: 19.04.2012 IT RM20120169
(43) Date of publication of application: 25.02.2015
(73) Proprietor: Consiglio Nazionale Delle Ricerche, 00185 Roma (IT)
(72) Inventor: MATTEINI, Paolo, I-00185 Rome (IT); RATTO, Fulvio, I-00185 Rome (IT); PINI, Roberto, I-00185 Rome (IT)
(74) Representative: Currado, Luisa
(86) International application number: PCT/IB2013/053044
(87) International publication number: WO 2013/156944

(56) References cited:
- WO-A1-2005/077330
- US-A1- 2002 192 280
- US-A1- 2011 104 052
- RUEL-GARIEPY E ET AL: "Thermosensitive chitosan-based hydrogel containing liposomes for the delivery of hydrophilic molecules", JOURNAL OF CONTROLLED RELEASE, ELSEVIER, AMSTERDAM, NL, vol. 82, no. 2-3, 21 August 2002 (2002-08-21), pages 373-383, XP004374935, ISSN: 0168-3659, DOI: 10.1016/S0168-3659(02)00146-3
- PAOLO MATTEINI ET AL: "Chitosan Films Doped with Gold Nanorods as Laser-Activatable Hybrid Bioadhesives", ADVANCED MATERIALS, vol. 22, no. 38, 8 October 2010 (2010-10-08), pages 4313-4316, XP055047249, ISSN: 0935-9648, DOI: 10.1002/adma.201002228
- PAASONEN ET AL: "Gold nanoparticles enable selective light-induced contents release from liposomes", JOURNAL OF CONTROLLED RELEASE, ELSEVIER, AMSTERDAM, NL, vol. 122, no. 1, 22 August 2007 (2007-08-22), pages 86-93, XP022208580, ISSN: 0168-3659, DOI: 10.1016/J.JCONREL.2007.06.009

## Description

### STATE OF ART OF THE INVENTION

### 1. Field of the invention

The present invention as defined by the claims relates to a matrix and a device for optically-controlled release of chemical species, based upon the light excitation of plasmonic nanometric particles developing heat in a controlled and localized way, wherein chemicals are contained in supramolecular aggregates of amphiphilic molecules.

The chemical species to be released can be a drug or in general any substance to be administered for therapeutic, diagnostic and/or cosmetic use. However, the invention is not limited to such fields as it can be applied in any field wherein a localized and controlled delivery of a chemical species usable in the present invention is requested.

The supramolecular aggregates of amphiphilic molecules containing the chemical species to be delivered are of thermosensitive type, that is they are able to modify some of their chemical and/or structural properties in presence of a temperature variation, thus determining the partial or total delivery of the chemical species contained therein.

### 2. Description of the state of art

Different solutions for using supramolecular aggregates of amphiphilic molecules, in particular micellar structures and liposomes, used to contain and transport hydrophobical and hydrophilic chemical substances are known.

For example, the article of HuaWei, Si-XueCheng, Xian-Zheng Zhang and Ren-XiZhuo Thermo-sensitive polymeric micelles based on poly(N-isopropylacrylamide) as drug carriers, Progress in Polymer Science 34 (2009) 893-910 describes different types of micelles which are designed to contain chemical species such as a drug and which perform a delivery of such chemical species due to the effect of the temperature rise above the lower critical temperature of some amphiphilic precursors constituting them. However, the same authors state that the mechanisms for releasing drugs contained in micelles require a very accurate local adjustment of temperature, which constitutes a critical element for the application thereof.

On the contrary, the fact of including drugs in micellar structures is described as from the European patent application Nr. 721,776, which however does not say anything about the localized administration of the carried drugs, as well as in the European patent applications Nr. 1392254, Nr. 1418945 and Nr. 1487892, in the US patent applications Nr. 2006/0216342 and Nr. 2010/0203150 e in the International patent application Nr. 2005/087825. Furthermore, the oral administration of liposomic or proliposomic structures is known, incorporating one or more drugs, as described for example in the International patent application Nr. WO 2006/062544. In these documents, the possible thermosensitivity of these supramolecular structures is not taken into consideration and the chemical species is not administered in localized way.

US patent application No. 2002/1012298 describes different types of liposomes able to incorporate a drug which can be delivered in the human body by means of a local hyperthermia artificially induced by means of localized heating at the site to be treated, or thanks to a local rise of the body temperature, caused for example by fever or inflammation of the tissues, with delivery temperatures ranging from 38°C and 45°C. Liposomes of the same type are also described in the International patent application Nr. WO 2009/062398.

In absence of a natural hyperthermia, the artificial induction of a localized hyperthermia can be implemented with different heating systems which can provide the local administration of radiofrequencies, microwaves, infrareds, but with a procedure which always results to be uncomfortable, invasive and potentially dangerous for the adjacent tissues.

US patent application No. 2007/0077230 describes a device for the controlled administration of liposomes and other thermosensitive nanostructures containing a drug, that is able to release the drug contained therein thanks to a local temperature rise. The device is able to implement an intratumoural injection of the nanostructures through a needle and a localized heating obtained with an antenna operating with radiofrequencies and microwaves. The antenna and the needle can be incorporated in the same administration device.

Although this solution tries to guarantee an exact localization of delivery of the closed molecular structures, both the administration procedure and the heating procedure result to be particularly invasive.

On the contrary, US patent Nr. 6623430 describes a device for obtaining the *in situ* disintegration of liposomes containing a drug, using an acoustic transducer able both to heat locally the application site and to implement a display of the generated thermal profile.

Another document which describes the administration of ultrasounds is the patent application Nr. 2005/0003008, wherein the drug is included in micellar structures which are invested and dissolved by means of the application of a flow of ultrasounds.

This energy form causes the vibration of the amphiphilic molecules constituting the micellar structure; therefore, it is wholly analogous to a heating form. However, the treatment does not involve solely the micelles but any tissue and biological component placed at said micelles, causing possible discomfort and possible damages at the local molecular level.

The International patent application Nr. WO 2011/001351 describes structures formed by amphiphilic molecules such as micelles, liposomes, nanospheres, polyimerosomes containing inside chemical species to be delivered for therapeutic purposes. The delivery takes place by means of the disintegration or dissolution of such structures, which is induced thanks to an external stimulus which can include the use of high intensity focalized ultrasounds, high intensity radiofrequencies, high frequency variable magnetic fields, hyperthermia induced by means of radiofrequencies, microwaves and so on. The suggested administration method also provides to include in said structures a contrast agent, that is a certain number of iron-, nickel-, cobalt-, zinc-based magnetic particles, in order to allow their localization by means of techniques for visualization of magnetic particles, in order to apply correctly one of said external stimuli. It is to be noted that such magnetic particles, once the administration is ended up, remain in the body together with the delivered chemical species.

US patent application Nr. 2009/0004258 describes a system for releasing drugs contained in thermosensitive liposomes wherein particles of paramagnetic material, such as iron oxide, are included; they cause a localized heating of 2-3°C if invested by a cyclic magnetic field, thus causing the drug delivery.

US patent application Nr. 2009/0054722 describes a solution analogous to the previous one, wherein heating is induced by a high frequency magnetic field. Even in these two last cases, once the thermosensitive liposomes containing the paramagnetic particles are broken down, these are delivered into the body.

In US patent application Nr. 2011/0230568 several types of thermosensitive polymeric structures are proposed, which can include a chemical species, by coupling them with a wide range of possible elements which can provide heat thereto, among thereof nanoantennas apt to be illuminated with a light beam. However, neither the problem of being able to obtain a localized delivery effect of the chemical species to be delivered, nor the problem of being able to dose the chemical species to be delivered, nor the problem of being able to control the temperature necessary to induce the delivery below a certain threshold, nor the problem of being able to localize the thermal effect are dealt with.

In all these cases, however, is difficult implementing an exact positioning of the structures containing the chemical species to be delivered at the level of the biological target to be treated, whether they are micelles, liposomes or other. Consequently, the administration of the chemical species results to be poorly localized and scarcely controllable.

Furthermore, in the mentioned cases wherein the release device also provides the presence of heat-generating particles such as paramagnetic particles or nanoantennas, the possibility of a whole removal thereof at the end of the treatment is not provided. Therefore they can remain in the administration target site and, more generally, with possible toxic effects in case of poorly biocompatible particles.

US2011/104052 discloses polymeric or hydrogel structures comprising heat-sensitive liposomes for drug burst release upon laser activation. The heating of said structure causes liposomes disintegration leading to the rapid release of the drug, consistent with the concept of burst release which is a rapid release of drug wherein the blood concentration of drug rises quickly and briefly plateaus and not with the concept of sustained or extended release.

WO2005/077330 discloses core-shell materials for controlled release of chemical compounds induced by near infrared (NIR) light. The chemical compounds are retained in the core while the shell comprises a NIR light absorber. When said core-shell material undergoes to electromagnetic excitation by means of NIR light, the shell breaks down allowing the immediate and non-controlled release of the chemicals from the core. The release from the core-shell particle is irreversible since said structure is completely destroyed at the end of the excitation.

Matteini P., et al., 2010, "Chitosan films doped with gold nanorods as laser-activatable hybrid bioadhesive", Advanced Materials, 22(38), 4313-4316 disclose a device made of chitosan film comprising gold nanorods, where laser excitation of the nanorods with single-pulses in the millisecond timescale produces temperature values of about 130 °C, which generates melting of selected areas of the chitosan film which ultimately can adhere to a biological tissue. Said film cannot be used for drug controlled-release since it is not thermosensitive at temperature values that are considered safe and useful for controlled drug delivery (i.e. <<130°C and > 37°C), is not porous, the above-cited melting is not reversible and, differently from liposomes and micelles, its chemical nature impedes the retaining of the most part of drugs and of other chemical species of interest for administration at physiological temperature. US2002/0192280 discloses Paclitaxel in nano-sized micellar carriers of diblock copolymers loaded into cross-linked polymeric films of chitosan.

### SUMMARY OF THE INVENTION

The technical problem which the present invention proposes to solve consists in overcoming the drawbacks mentioned with reference to the state of art.

In particular, a main object of the matrix and device according to the invention as defined by the claims is to allow a controlled release and localized administration of a chemical species, by exploiting the thermosensitivity of the structures of amphiphilic molecules used as reservoir thereof, leading to a localized and safe administration without requesting a direct heating of the application area or however an invasive, discomfort procedure or potentially dangerous for living tissues adjacent to the application area. Another main object of the present invention is to allow a release which is controlled in the space and during time so that the administration could be put under the total control of an operator, even subsequent to the application of the matrix and device in the seat to be treated with the chemical species to be delivered. Highly preferably is to perform, with the same matrix and device positioned in its administration seat, both partial deliveries and deliveries which can be repeated on a time span without this requesting the application of a new matrix and device.

Furthermore, one wants to guarantee the possible, if requested, complete removability of the matrix and device once the administration has taken place. Additionally, one wants to ease the handling of the structures containing the chemical species to be delivered and of the heat-generating particles, that is of the matrix and device containing them until its final positioning.

The technical problem of obtaining controlled release of active ingredients covers three different aspects: controlling the releasing time when the device undergoes to light excitation; controlling the spatial spread of the released active ingredient and controlling the dosage of the released active ingredient.

The releasing time is influenced by the light excitation; the spatial spread of the released drug is firstly determined by the dimension of the incident light beam and of the illuminated area size and secondly by the heat-induced temporary hyperpermeability of cellular entities placed close to the illuminated area of the matrix..

The dosage of the released active ingredient depends on the illumination time and the light intensity.

The above-mentioned problem is solved by a porous polymeric matrix as defined by the claims transparent to a light flux in the visible or NIR spectrum comprising plasmonic nanometric particles absorbing light in the visible and NIR, and suprarmolecular aggregates of amphiphilic molecules containing chemical species, wherein said nanometric particles have a substantially homogeneous distribution within said porous polymeric matrix and said suprarmolecular aggregates of amphiphilic molecules are dispersed in and constrained to said porous polymeric matrix.

And by a release device as specified above, comprising:
- a porous polymeric matrix having pores with size so as to allow the passage of a chemical species to be delivered, said matrix being chosen so as to be substantially transparent to a light flux in the visible or NIR spectrum;
- a plurality of nanometric particles dispersed in said porous polymeric matrix with a substantially homogeneous distribution, apt to be excited when are invested by said light flux in order to generate heat, said heat-generating particles being dispersed and constrained to said porous polymeric matrix; and
- a plurality of thermosensitive structures under the form of suprarmolecular aggregates of amphiphilic molecules, containing said chemical species to be delivered at a predetermined administration temperature, said thermosensitive supramolecular structures being dispersed and constrained to said porous polymeric matrix,
wherein said nanometric particles and said thermosensitive supramolecular structures of amphiphilic molecules are distinct to each other following to different dispersions,
the nanometric particles being apt to increase the average temperature of the porous polymeric matrix at said predetermined administration temperature when the device is illuminated by a light flux at a predetermined radiation intensity, the nanometric particle dispersion being chosen to not affect the structural integrity of the porous polymeric matrix at said predetermined administration temperature.

The above combination of components, constituting the essential distinguishing features of the present invention, allows a controlled release of active ingredients in time, space and dosage.

This means that the dosage of the active ingredient can be controlled in consecutive administrations by means of the same matrix and device which remain intact and does not deteriorate upon light and heat treatment.

A further object of the present invention is the use of the above porous polymeric matrix for controlled delivery of chemicals, active ingredients, drugs, such as anticancer agents.

The porous polymeric matrix can be easily moulded in a thin film and it can be adhered at the area wherein one wants to implement the delivery of the chemical species.

Preferably said thin film has a thickness comprised between 10 µm and 1000 µm.

Preferably the pores within said porous polymeric matrix have sizes comprised in the range of 10 nm ÷ 5000 nm.

Preferably said porous polymeric matrix is a hydrogel, more preferably a hydrogel comprising chitosan.

The chemical can be an active ingredient such as a drug, preferably an anti-tumour drug.

Said film can be used for cutaneous or subcutaneous administration.

The film can be adhered at cutaneous or subcutaneous level, but it can be even deposited on a site inside the body, for example to be subjected or subjected to surgical treatment.

Preferably the polymer constituting the matrix is chemically stable in physiological environment to allow a removal of the device in its integral form, or on the contrary it is biodegradable in physiological environment in a period subsequent to the in situ application. The plasmonic nanometric particles are metallic, apt to be excited at determined frequencies of plasmonic resonance if illuminated by a light beam, more preferably in the shape of nanorods, most preferably are gold nanorods.

When said plasmonic nanometric particles are gold nanorods the gold concentration inside the gold nanorods is preferably between 0.2 mM and 0.8 mM.

Preferably said plasmonic nanometric particles have sizes and an aspect ratio so as to be excited at determined frequencies of plasmonic resonance when illuminated by a light flux with wavelength comprised in the range of 500 ÷ 1200 nm.

Preferably said nanometric particles have preferential sizes between 20 nm and 120 nm of length and 5 nm and 30 nm of diameter.

Preferably said plasmonic nanometric particles inside said porous polymeric matrix under hydrated form are comprised in the range of 0.0001 ÷ 1 wt% and preferably 0.001 ÷ 0.1 wt%.

Preferably the minimum distance between gold nanorods is equal or higher than 2.5 times the diameter thereof.

Optionally said plasmonic nanometric particles are coated by an organic material, preferably polyethyleneglicole (PEG) or inorganic material, preferably silica, or titania. The supramolecular structure is a micellar structure. Preferably said supramolecular structure is constituted by block copolymers.

Preferably said supramolecular structure comprises polycaprolactone (PCL) and polyethylene oxide (PEO).

Another object of the present invention is a method for the preparation of the above porous polymeric matrix by deposition of an aqueous solution of the polymer at acid pH and subsequent solvent evaporation at a temperature of 20 ÷ 35 °C.

Preferably the solvent evaporation is terminated by producing matrix insolubilization by means of alkalinisation for a time between 1 and 30 minutes, followed by one or more passages of neutralization in water, before the complete evaporation of the solvent takes place, by leaving to pass a time comprised between 30 minutes and 6 hours from the deposition. This causes free polymer strands to form a cross-linked polymer matrix with a porous structure.

The invention also provides chemicals for use in delivering to a patient in need thereof comprising adhering a porous polymeric matrix transparent to a light flux in the visible or NIR spectrum comprising nanometric particles absorbing light in the visible and NIR, and suprarmolecular aggregates of amphiphilic molecules containing chemical species, wherein said nanometric particles have a substantially homogeneous distribution within said porous polymeric matrix and said suprarmolecular aggregates of amphiphilic molecules are dispersed in and constrained to said porous polymeric matrix shaped in the form of a film, in a defined area at cutaneous level or subcutaneous level or on a site inside the body by means of surgical treatment and subjecting said film to irradiation by means of a light source in the visible or NIR spectrum.

The nanometric particles are of metallic type, apt to be excited with light radiation, advantageously proposed under the form of nanorods, preferably made of gold with predefined sizes and shape, so that the excitation thereof can take place at a particular electromagnetic wavelength comprised in the spectral window of the visible and NIR spectrum.

In particular, the light flux could be generated with a wavelength comprised between 500 and 1200 nm, including the so-called therapeutic window, wherein the light has the maximum penetration inside the biological tissue. In this way, the release device could be illuminated directly from outside the body even if arranged in subcutaneous position, to allow the quick inflow of the chemical species in the area close to the device application area.

The light flux could be provided under the form of a low intensity laser beam or through the emission of a LED, both not interfering with the tissues or with the polymeric matrix.

The excitation of the plasmonic resonances (that is of the collective oscillations of electronic charges at characteristic frequencies forced by the oscillating electric field of the incident light) of the metallic nanoparticles generates a heat quantity due to the photothermal effect (that is by conversion of radiation into heat thanks to dissipative effects in the oscillating electric charge under the forcing action of the light electric field). Such heat develops directly inside the release device, without requesting a heating of conductive type from outside. Said photothermal effect can be modulated depending upon the light intensity (that is the light power per surface unit) used in the illumination time, determining a local heating of variable entity and duration. For example, such photothermal effect can be kept below those temperatures causing irreversible heat damages to the tissues adjacent to the device.

For applications inside the body, wherein it is not possible illuminating the device from outside, the light flux could be provided by an optical fibre inserted in the body by percutaneous or endoscopic way so as to implement a controlled administration, at the site to be treated, and with the necessary dosage.

The supramolecular aggregates of amphiphilic molecules constitute a delimitated structure which can contain a wide range of chemical species which can be delivered with the present device. In particular a micellar structure could contain a chemical species scarcely or not soluble in water, whereas a liposome could contain even a hydrophilic chemical species. With micellar structures, it is possible exploiting the property of some types of micelles of contracting or swelling if the temperature is raised, that is if the temperature deviates even by few degrees from a characteristic transition temperature. The volume variation can stimulate the expulsion of the chemical species encapsulated in each single micellar structure.

In case of other structures constructed by amphiphilic molecules, no longer under the scope of the invention, such as liposomes or other, the temperature local rise can produce a temporary weakening of the intermolecular chemical links, which make such structures temporary permeable, with controlled discharge of the contained chemical species.

The restoring of the initial temperature, once the light flux has ceased, brings back said structures in the initial configuration. These can possibly contain a residual of chemical species which can be delivered later. In each case, both such structures and the heat-generating nanometric particles remain, during and after the delivery, included inside the porous polymeric matrix and they are not then delivered together with the chemical species.

### BRIEF DESCRIPTION OF THE DRAWINGS

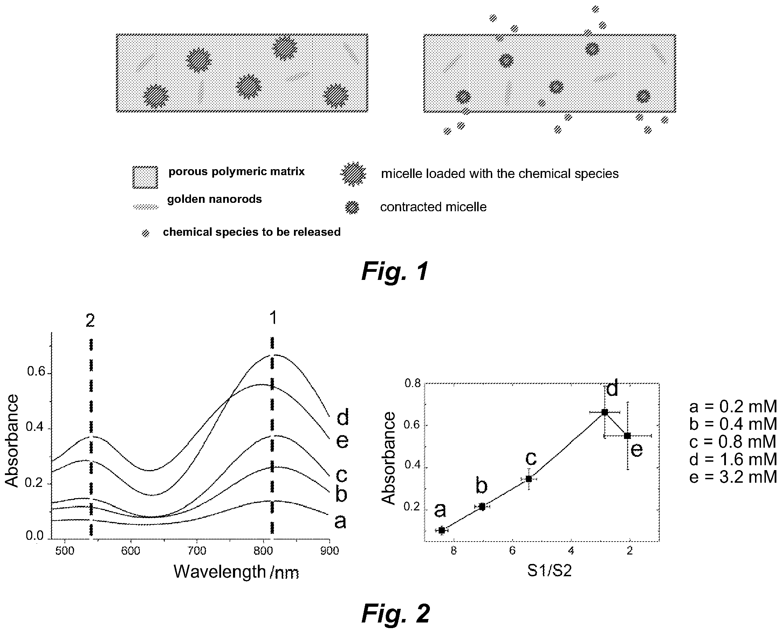

Figure 1 illustrates a scheme of a device for releasing chemical species, wherein the polymeric matrix contains nanometric metallic particles, briefly called nanoparticles, such as for example nanorods made of gold which can be excited in the NIR, and thermosensitive supramolecular aggregates of amphiphilic molecules, for example thermosensitive micelles containing a chemical species, and wherein the heat generated by the nanoparticles activates a structural modification of such aggregates leading to the controlled delivery of the chemical species in the surrounding environment.
Figure 2 in the left diagram illustrates the optical absorption spectra of polymeric matrixes of chitosan (3.5 % w/v) doped with nanorods made of gold with an aspect ratio (ratio between height and diameter of the nanorod) of about 4 and containing polycaprolactone-polyethylene oxide-polycaprolactone (PCL-PEO-PCL) micelles (10 wt%) with different density of nanorods expressed as concentration of contained gold (a = 0.2 mM; b = 0.4 mM; c = 0.8 mM; d = 1.6 mM; and = 3.2 mM); in the diagram on the right the variation in the ratio of the area of absorption band 1 (S1) in the near infrared (λ₁ ≈ 810 nm) and of the band 2 (S2) in the visible (λ₂ ≈ 540nm), characteristic of the nanorods, as a function of the maximum absorption value of the band 1, for different gold concentrations;

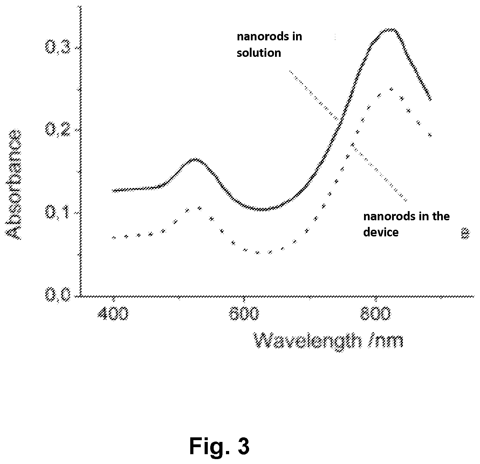

Figure 3 shows a diagram which illustrates the matching between the absorption spectra of gold nanorods in solution (continuous line) and the absorption spectra of a matrix of chitosan doped with gold nanorods with an aspect ratio of about 4 and a gold concentration equal to 0.8 mM containing micelles of PCL-PEO-PCL type (dotted line).

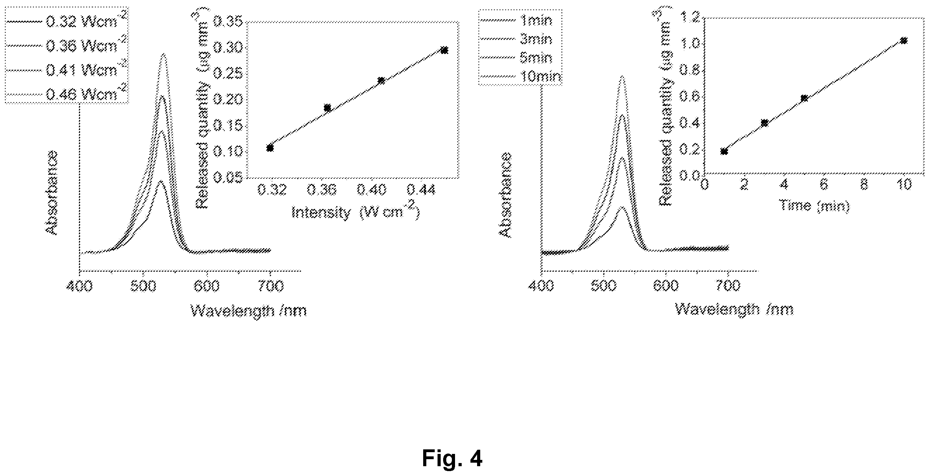

Figure 4 comprises some diagrams illustrating the absorption spectra of rhodamine 6G delivered in physiological solution (PBS, pH 7.4) by matrixes of chitosan (3.5 % w/v) doped with gold nanorods with an aspect ratio of about 4 and with a gold concentration of 0.8 mM, containing micelles of PCL-PEO-PCL type (10 wt%) and 1% w/v of rhodamine 6G: on the left, upon varying the light intensity of an 810 nm AIGaAs diode laser after 1 minute of illumination in continuous irradiation mode (cw); and on the right, upon varying the illumination time at a fixed intensity of 0.36 W/cm². The small squares show the values of rhodamine 6G delivered in a solution volume (expressed as µg/mm³) upon varying the above-mentioned parameters;

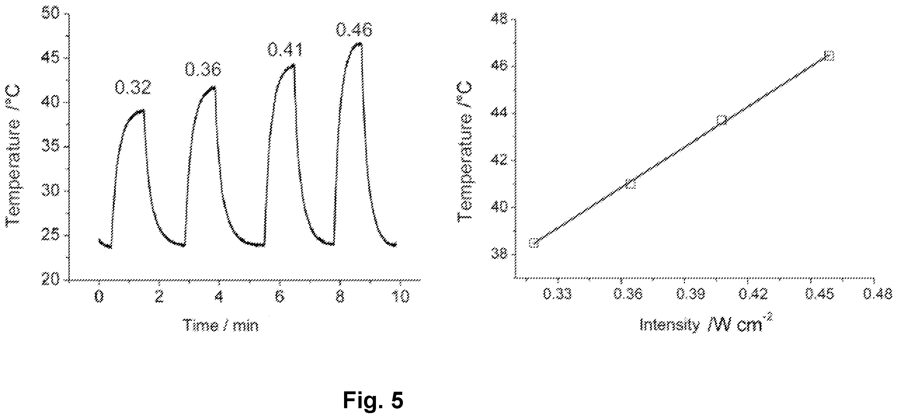

Figure 5 in the diagram on the left illustrates the temperature profiles measured upon varying the light intensity (0.32 - 0.36 - 0.41 - 0.46 W/cm²) of an AIGaAs laser with emission at 810 nm and continuous irradiation mode (cw) for light stimuli of 1 minute on polymeric matrixes of chitosan (3.5% w/v) doped with gold nanorods with an aspect ratio of about 4 and a gold concentration of 0.8 mM containing micelles of PCL-PEO-PCL type (10 wt%), dipped in physiological solution (PBS, pH 7.4); the diagram on the right displays a linear variation of the maximum temperature measured at the level of the matrix with the fixed laser intensity , ;

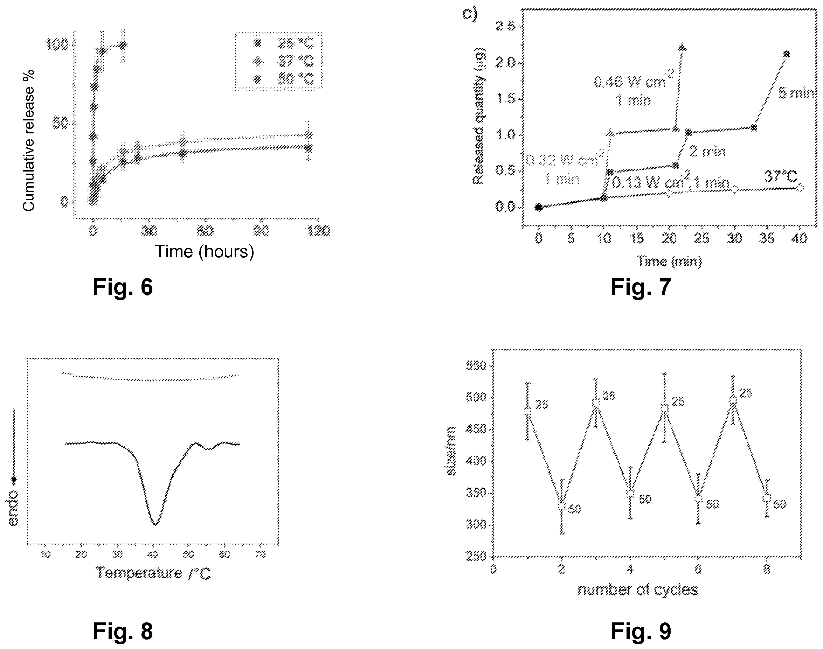

Figure 6 shows a diagram which illustrates the cumulative release of rhodamine 6G in PBS (pH 7.4) from a device analogous to the one described in Figure 4 once subjected to different temperature values produced by a heating stage. The buffer solution surrounding the sample was collected and replaced with fresh buffer after each period of treatment. Error bars are based on the SD of triplicate samples.
Figure 7 shows different tests of rhodamine 6G release from a device analogous to the one described in Figure 3 in accordance with different combinations of illumination times and light intensities, namely: small triangles (**▲**) 10 min at 37 °C, 1 min at 0.32 W cm⁻², 10 min at 37 °C, 1 min at 0.46 W cm⁻²; small quares (■)10 min at 37 °C, 1 min at 0.13 W cm⁻², 10 min at 37 °C, 2 min at 0.13 W cm⁻², 10 min at 37 °C, 5 min at 0.13 W cm⁻²; small rhombus (0) ground release at 37 °C.
Figure 8 shows a diagram which illustrates a differential scanning calorimeter analysis of a polymeric matrix of chitosan doped with gold nanorods and containing micelles of PCL-PEO-PCL type (continuous line), compared to the one obtained on an analogous system, but without micelles (dotted line), at the scanning speed of 5°C/min;
Figure 9 shows a diagram which illustrates the average size of micelles of PCL-PEO-PCL type (aqueous solution containing 1 mg/mL of PCL-PEO-PCL copolymer) evaluated by means of Dynamic Light Scattering (DLS) measurements upon varying the temperature between two prefixed values of 25 °C (up) and 50 °C (down) on various measurement cycles;

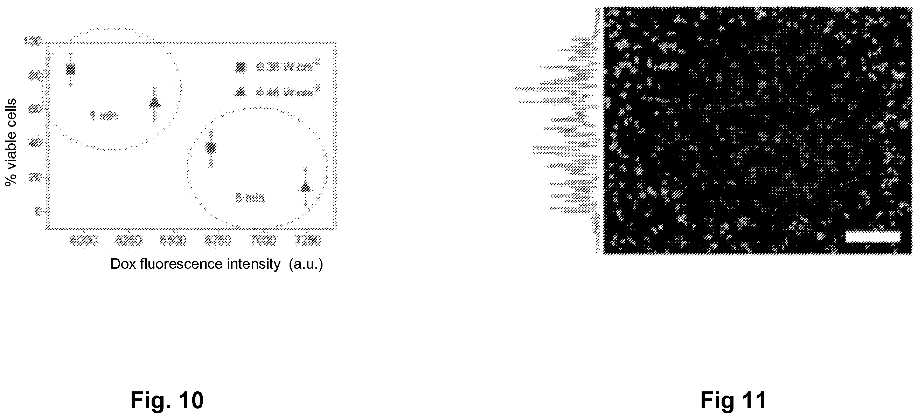

Figure 10 shows a diagram which illustrates the decrease in HeLa cell viability (expressed as % calcein-positive cells averaged over 4 samples ± SD) caused by Doxorubicin (Dox) release from a device analogous to the one described in Figure 4 (except for the replacement of rhodamine 6G with 1mg/mL Dox) at different laser intensities (0.36 W cm⁻² and 0.46 W cm⁻²) and irradiation times (1 min and 5 min) as a function of internalized Dox (evaluated as intracellular Dox fluorescence, n=4).
Figure 11 shows the spatial control over Dox release by irradiating a 2 mm area of a sponge (0.46 W cm_2, 5 min) placed in contact with HeLa cells: Dox-induced cell death is confined to those cells in close proximity with the irradiated portion of the sponge (bar ¼ 500 mm) (the confinement of the PI stain is emphasized by the profile on the left).

### DETAILED DESCRIPTION OF THE INVENTION

The context wherein the present release device according to the invention has been conceived is that of releasing in a controlled way chemical species activated by an external light stimulus in the visible or NIR spectrum.

In general, the present invention as defined by the claims relates to the development of a device able to implement the release of one or more chemical species, triggered by a light stimulus. More in particular, the present invention relates to the development of a device implantable inside the body or applicable upon the cutaneous surface or in other biological environment such as a cellular culture, an explanted tissue or an artificial tissue, for releasing chemical species by the activation with a light beam emitted by a laser device or LED in the visible or in the NIR spectrum, which can be used for example in the field of medical therapies requesting to provide a drug in a localized and controlled way in time. However, it is meant that such release could be useful even for not medical purposes, in a different context from the biological one.

The above-mentioned release device as claimed is constituted by a porous polymeric matrix, doped with a dispersion of nanoparticles, in particular metallic nanoparticles, which carry out the function of absorbers with the property of converting light into heat under illumination in the visible or in the NIR spectrum. An example of nanoparticles is represented by the gold nanorods.

The heat generation, in turn, activates the release of molecules of a chemical species loaded inside thermosensitive supramolecular aggregates of amphiphilic molecules; such aggregates being contained inside the polymeric matrix.

More in detail, the release is implemented by sending on such device on a portion thereof a light radiation which is absorbed by the metallic nanoparticles contained in the device, generating a localized and controlled temperature increase which, in case of *in vivo* applications, will be slightly above the physiological temperature and in any case below the limit of thermal damage to tissues and cells. Such heating of the device or a portion thereof activates a characteristic thermal transition of the aggregates of amphiphilic molecules with volume variation of the same and/or of the permeability thereof or however of some chemical and/or structural properties thereof, thereafter the chemical species is released in the surrounding environment through the pores of the polymeric matrix.

The release effect triggered by light radiation is reversible and repeatable in subsequent times and can be modulated depending upon the light intensity and upon the exposure time to the light stimulus. The choice of nanoparticles with absorption properties in the NIR spectrum (700-1200 nm) allows using more penetrating light sources inside the body (as far as some cm). Such sources are compatible with commercial systems, including LEDs and lasers.

This release device as claimed allows obtaining a controlled release of chemical species, based upon a photothermal effect, from a porous polymeric matrix previously loaded with such species and containing a dispersion of metallic particles. Differently from the previous systems, the temperature rise, generated by the conversion of the light flux into heat thanks to the metallic particles, and the consequent release effect, can be highly controlled depending upon the used light intensity and the exposure time to the light stimulus. This aspect provides an accurate and precise control system of the released dose.

By referring to Figure 1, the release device comprises at least four components.

The first one thereof is constituted by a porous polymeric matrix with pores with such a size so as to allow the passage and the discharge of a chemical species to be delivered. The used material to implement this matrix, according to a preferred embodiment, is a hydrogel, that is a structure constituted by a conglomerate with high content of water and of polymeric nature. It is porous with pores comprised in the range of 10 nm ÷ 5000 nm, and preferably between 50 and 500 nm.

In one type of application, the material constituting the porous matrix is a hydrogel with biodegradability features on medium-long time (months) under physiological conditions; a material having these features is chitosan, a linear polysaccharide constituted by D-glucosamine and *N*-acetyl-D-glucosamine, obtained by deacetylation of chitin, generally coming from the exoskeletons of crustacea. Possible alternatives are other polysaccharides such as alginate, derivatives of cellulose (carboxymetylcellulose, metylcellulose etc.), dextran, guar rubber, etc., as well as gels based upon acrylic acid (Carbopol® etc.). However, the possibilities are not limited to these examples.

These formulations are particularly suitable for implants inside the body, apart that their use is not limited to medical applications. The material constituting the hydrogel has then the following features: it is insoluble in aqueous solution at physiological pH and for temperatures lower than 60°C, it can be modelled in different forms in the manufacturing phase; it is able to be prepared in a porous form and it is not subjected to thermal and structural modifications in the involved temperature range for releasing the chemical species, and however for temperatures lower than 60°C. In an additional version of the invention, the hydrogel itself can have pharmacological effects, such as for example, anti-bacterial and anti-inflammatory effects.

The release device is a hydrogel structure preferably planar, implemented in the shape of film with a variable size depending upon the use conditions and with a thickness comprised between 10 µm and 1000 µm and preferably between 40 µm and 500 µm. The hydrogel matrix, in the hydrated form, is then flexible and easily adaptable to irregular and non-planar surfaces. For implantations inside the body the device can be fastened to the surface of a tissue by using sutures or other surgical devices. For implantations outside the body (for example skin) the device can be fastened by using plasters, gauzes or bandages.

By way of example, a porous polymeric matrix made of chitosan can be prepared by deposition of an aqueous solution at acid pH (in particular at a pH between 2 and 6) of chitosan on suitable moulds, with a subsequent evaporation of the solvent (water) preferably performed in the range 20 ÷ 35 °C. In order to obtain a matrix containing pores with previously specified sizes, a matrix insolubilization is induced by means of alkalisation, for example by using NaOH 1M for a period preferably comprised between 1 and 30 minutes, followed by one or more neutralization passages in water, before the complete solvent evaporation takes place, and however preferably by waiting a time comprised between 30 minutes and 6 hours from deposition.

The second one of said components, by referring to Figure 1, is constituted by a plurality of nanometric particles dispersed in the above-mentioned porous polymeric matrix with a substantially homogeneous distribution, apt to be excited when invested by said light flux, generating heat. These nanoparticles are metallic particles able to perform an optical response, that is light absorption and diffusion, which originates from the excitation of plasmonic resonances, that is a collective oscillation of electronic charges with characteristic features, forced by the oscillating electric field of the incident light.

Such resonances determine very high optical absorption coefficients, higher by several orders of magnitude than those of the common organic chromophores. The optical absorption then varies depending upon the type of metal, shape and sizes of the particles. For example, in case of golden nanoparticles, whereas particles with spherical shape typically show a single optical absorption peak around 540 nm, non-spherical gold particles such as gold nanorods typically show a main peak in the NIR matching the so-called and already mentioned "therapeutic window" (700 ÷ 1200 nm), characterized by a maximum light penetration inside the body. Therefore, the particles which are used in the present release device preferably are made of gold and have a cylindrical shape (gold nanorods) and preferential sizes between 20 nm and 120 nm of length and 5 nm and 30 nm of diameter. The absorption spectrum of these particles can be modulated so as to have a main peak at a given wavelength inside the therapeutic window; this is typically implemented by modifying the aspect ratio between height and diameter of the particles during their preparation phase. Such particles are homogenously dispersed inside the hydrogel and remain confined therein thanks to the interactions established with the porous polymeric matrix. Typical densities of the nanoparticles inside the matrix in the hydrated form are comprised in the range 0.0001 ÷ 1 wt% and preferably 0.001 ÷ 0.1 wt%.

Such confinement remains unaltered as long as the matrix keeps its integrity and therefore it depends upon the degradation process of the matrix itself, variable according to the external conditions whereunder it is, notwithstanding in many applications such matrix, that is the whole device, can be removed from the application site thereof.

The metallic nanoparticles are then excited with a light source and in particular with a laser or LED device, by generating a heat quantity in the surrounding matrix which first of all depends upon the used light intensity.

The heat development translates into a localized temperature increase and more precisely in a temperature gradient from a minimum of 0.5 °C to a maximum of 50°C, and preferably from a minimum of 3°C to a maximum of 23°C starting from an initial temperature of 37°C. The temperatures falls down once the excitation has ceased, depending upon the heat dissipation speed and therefore the thermodynamic features of the environment wherein the heat is dissipated. The particles keep unaltered the optical properties after the above-mentioned illumination, so that it can be repeated an undetermined number of times without losing efficiency.

The interactions between the hydrogel and the nanoparticles included therein are stable so as to keep the hydrogel/nanoparticles system in its original shape and structure even after the light illumination described above. Furthermore, the mechanical properties and in particular the mechanical stability of the porous polymeric matrix are not influenced by the light radiation and by the consequent inner generation of heat, nor after repeated light illuminations.

It is further meant that, for specific applications, the above-mentioned nanoparticles could be selected in alternative shapes and metallic compositions and they could be constituted even by non-metallic nanoparticles, for example carbon nanotubes, graphene or polymeric particles containing an organic chromophore with absorption properties in the visible or in the NIR spectrum. The choice of the type of nanoparticles to be used for implementing the device is guided by reasons of different nature such as working wavelength, photothermal transduction efficiency, toxicity, degradability, photothermal stability and affinity for the porous matrix.

The third one of said components of the release device according to the present embodiment is a plurality of thermosensitive structures in the shape of supramolecular aggregates of amphiphilic molecules, containing said chemical species to be delivered at a predetermined administration temperature. These structures are arranged so that they are dispersed in said porous polymeric matrix, with preferably homogeneous distribution, so as to be constrained to the matrix itself.

There is a wide range of supramolecular structures of amphiphilic molecules, that is containing both hydrophobic and hydrophilic functional groups. Examples of molecules with amphiphilic features are phospholipids, neutral and ionic surface-active agents, fatty acids, block copolymers, etc. The phospholipids and the block copolymers are particularly preferred. Examples of aggregates of amphiphilic molecules of the above-specified type are micelles, liposomes and polymerosomes.

The micellar structures or micelles form when, under conditions of temperature equal or higher than *Krafft* temperature, the concentration of the amphiphilic molecules reaches a critical level, designated as critical micellar concentration (CMC). At this critical concentration, the molecules aggregate by generating generally spherical supramolecular structures, but even other shapes such as cylindrical, lamellar or discoidal ones. In polar solvents, such as water, the hydrophobic portion of the amphiphilic molecule orientates inside the aggregate by forming the micelle core, whereas the hydrophilic portion orientates outside by constituting the micelle surface layer. The core, having a hydrophobic nature, can be used to include a substance not soluble, or poorly soluble in water, that is substantially lipophilic, which remains trapped inside the micelle.

Under liposome, instead, a usually spherical capsule or vesicle is meant, which is formed by phospholipids, which organize themselves to form a double-layered membrane in aqueous or polar environment. In the liposome centre then a cavity is formed which can be used to hold a compound soluble in water, that is a hydrophilic substance. Viceversa, the liposome walls can be used to confine a compound poorly soluble, or not soluble, in water, that is a hydrophobic substance.

Under polymersome a vesicle typically is meant, constituted by amphiphilic block copolymers, which can include an aqueous cavity, which can be used to hold a hydrophilic substance which can be released outside. Similarly to the liposomes, hydrophobic substances can be confined inside the polymerosome membrane.

The above-mentioned supramolecular structures, according to the present invention, are thermosensitive, that is they have the property of being subjected to a chemical and/or structural modification at a certain transition temperature which fosters a release of the substance contained inside thereof.

In case of micellar structures, according to the invention the property thereof of contracting or swelling if the temperature is raised, that is if the temperature moves even by few degrees from a characteristic transition temperature, can be exploited. The contraction can produce the expulsion of a chemical species encapsulated in each single micellar structure. Possible thermosensitive micellar structures which can be used in the device object of the present invention are: micelles containing poly(*N*-isopropylacrylamide) (PNIPAAm) such as hydrophobic or hydrophilic block in association with hydrophilic or hydrophobic blocks such as polyethyleneglicole (PEG) (or polyethylene oxide (PEO)), polymethylmetacrylate (PMMA), polycaprolactone (PCL), polystyrene (PS), poly(lactic-co-glycolic) acid (PLGA), polybutilacrylate (PBA) etc.; micelles containing PEG (or PEO) exposed on the outside and a core formed by blocks of polyaminoacids (PAA), polyaminoesters (PAE), polycaprolactone (PCL), polypropylene oxide (PPO), polybutylene oxide (PBO), etc.

In case of other supramolecular structures constituted by amphiphilic molecules, no longer in the scope of the invention such as liposomes or others, the temperature local rise can produce a temporary weakening of the intermolecular chemical bonds at a characteristic transition temperature, which makes the walls of such structures temporarily permeable, with controlled discharge of the included chemical species. Possible thermosensitive liposomic structures in the device object of the present invention include the following phospholipids or mixtures of these phospholipids: phosphatidiycholine (PC), phosphatidylglycerol (PG), phosphatidylserine (PS), phosphatidylinositol (PI) and derivatives thereof. However other different alternatives may exist. Liposomic structures can also include cholesterol. Liposomes may include PEG or be modified with thermosensitive polymers such as PNIPAAm or other polymers.

The restoring of the initial temperature, upon ceasing the light flux, sees said structures returning closed and integer, in case containing a residue of chemical species which can be released later. In any case, both the closed structures and the heat-generating nanometrical particles, during and after the release, remain integrated inside the porous polymeric matrix and they are not then released together with the chemical species, at least as long as degradation phenomena of said matrix do not take place.

The embodiment example of the release device described herein comprises micelles, constituted by block polymers with hydrophobic blocks and hydrophilic blocks. In particular, the polymer used for the thermosensitive micelles of the present embodiment is a copolymer with three blocks containing chains of polycaprolactone (PCL) and polyethylene oxide (PEO) of type PCL-PEO-PCL with molecular weights preferably of 500 ÷ 1000 Da and 1000 ÷ 2000 Da for each one of the two blocks of PCL and the block of PEO, respectively. These micelles are included inside the pores of the porous polymeric matrix and they are in thermal contact thereto. It is meant that the micelles can be inside the porous matrix under the insulated form or under the form of dimer or higher aggregate depending upon the concentration thereof and the tendency to aggregation thereof. The concentration of the supramolecular structures of amphiphilic molecules inside the release device has a maximum limit of about 70 wt% and it is preferably in the range of 5 ÷ 50 wt%.

However, the above-mentioned maximum limit substantially depends upon the risk of compromising the overall structure of the device porous matrix, whereas there is no minimum limit. An example of useful density can be in the range 10 ÷ 30 wt% of micelles..

Such micelles are thermosensitive, that is they can be subjected to a structural modification at temperature values above a determined characteristic transition temperature. The structural modification can consist in a variation the micelle average volume as for example a shrinking (contraction) with stimulation of the expulsion of molecules of a chemical species previously loaded inside, for example a pharmacological agent. The volume variation can be due to a change in solubility parameters of the blocks constituting the polymeric chain upon increasing temperature. The structural transition of the micelles preferably falls above 37°C and in particular above 38°C and below 60°C. The structural transition is reversible and therefore it can be induced repeatedly and in subsequent time for an undetermined number of times, and however not lower than ten times, without losing efficiency.

The chemical species, fourth component of the device according to the present invention, is loaded inside the supramolecular structures of amphiphilic molecules, that is in the present example in the micelles contained in the polymeric matrix.

The chemical species can be for example a pharmacological agent, in particular an antitumor pharmacological agent. Other examples of chemical species which can be loaded in the closed structures of amphiphilic molecules are for example: anaesthetics, anti-inflammatory drugs, antibiotics, antiviral agents, analgesics, antidepressant drugs, anticoagulants, diuretics, anticholinergics, vasodilators, drugs for the hypertension, sedatives, drugs for treating chronic diseases such as diabetes, cardiovascular problems, neurodegenerative problems, autoimmune diseases, Parkinson, Alzheimer, etc. The chemical species which can be loaded can be hormones, antibodies, proteins, glycoproteins, lipoproteins, polysaccharides, nucleic acids, organometallic compounds, etc. Other chemical species which can be loaded can be: colorants, flurophores, photosensitizing agents and contrast agents to be used in several fields such as for example the aesthetical one, apart from the biomedical and the pharmacological one.

By using micelles, the chemical species which can be loaded is poorly soluble in water, shows poor chemical affinity for the porous polymeric matrix and high affinity for the hydrophobic core of the micelles. The chemical species can be delivered by the micelle in the surrounding environment after an increase in temperature inducing a structural modification in the micelle as described above. The increase in temperature, in turn, is produced by the conversion of a light stimulus into heat, mediated by the optical absorption of metallic particles dispersed in the porous matrix therewith the micelles are in thermal contact. The increase in temperature allows reaching then a characteristic administration temperature thereat the chemical species is partially or wholly expelled by the structure of amphiphilic molecules.

The release of the chemical species by the release device can be modulated depending upon the radiation parameters, in particular the light intensity and the exposure time to radiation. Such parameters allow raising the temperature, first of all, of the environment wherein the structures of amphiphilic molecules are, up to a value corresponding to the administration temperature, and to control the duration of exposure at such temperature. The administration temperature is identified by an efficient intensity of the light flux exciting the nanoparticles dispersed in the matrix without influencing the mechanical stability, as well as the structure and the shape of the porous polymeric matrix without inducing irreversible thermal damages to the surrounding biological structures, but however sufficient to activate a characteristic thermal transition of the structures of amphiphilic molecules so that a release of the chemical species included therein can be induced.

Upon going into more details in the present embodiment example, the release device described herein is able to keep unaltered the chemical-physical features and the features of activated release from light for at least 30 days, if kept in water or in a physiological buffer (e.g. PBS, pH 7,4) at the temperature of 4°÷ 10°C.

The properties of optical absorption, in particular in the NIR, of the release device described herein depend almost exclusively upon the presence of the metallic nanoparticles (as the other components of the device absorb only weakly in the visible and in the NIR). It is possible to vary the density of the metallic nanoparticles so as to obtain an optimum homogeneous dispersion thereof inside the porous polymeric matrix and therefore an efficient and reproducible photothermal effect.

By referring to Figure 2, the absorption spectra of matrixes of chitosan (3.5% w/v) are shown. These matrixes contain gold nanorods with an aspect ratio of about 4 at a different density of particles expressed as gold concentration, and micelles of PCL-PEO-PCL (10 wt%) described above: the curves are identified as (a), (b), (c), (d) and (e) with the following gold concentrations: a = 0.2 mM; b = 0.4 mM; c = 0.8 mM; d = 1.6 mM; e = 3.2 mM. Furthermore, even the variations in the ratio between the area of band 1 (S1) in the near infrared (λ = 810 nm) and of band 2 (S2) in the visible (λ = 540nm) depending upon the maximum absorption value of band 1 are represented. A decrease in the ratio S1/S2 suggests a deterioration of the optical properties of the device.

It must be noted that formulations with low-average concentrations of gold i.e. in the range 0.2 ÷ 0.8 mM (curves a ÷ c) represent a compromise solution between an adequate capacity of optical absorption, and therefore of photothermal conversion, and a substantially homogeneous distribution of the particles inside the matrix. For higher concentrations, that is higher than 0.8 mM of gold (curves d - e), substantial aggregation effects due to the close distance among particles take place, which favour plasmonic coupling effects, with consequent widening and decrease in intensity of the spectral absorption band in the NIR, which translates into a poor control on the optical response and into a loss in the photothermal conversion efficiency of the device. This effect is clearly displayed in the diagrams of Figure 2.

Therefore, being able to keep the nanoparticles at sufficient distance therebetween is essential to guarantee both the efficiency and the reproducibility of the photothermal process and therefore of the release of the chemical species. The plasmonic coupling effect takes place over distances smaller than 2.5 times the particle diameter and it has been studied in particular for gold nanoparticles with absorptions in the NIR, such as nanorods, silica/gold *nanoshells*, *nanocages* and *nanoframes.* A possible variant, to obviate the problem of the plasmonic coupling in case of high concentrations of particles, is to use organic coating materials such as PEG or inorganic materials such as silica, or titania in order to keep the physical separation equal or larger than the above-mentioned values. The possibility of homogenously dispersing the nanoparticles in the polymeric matrix, apart from optimizing the system absorption as discussed above, also allows to obtain a homogenous temperature distribution inside the release device and therefore a much more controllable release effect. All the supramolecular structures of amphiphilic molecules on average will be subjected to such increase in temperature and therefore they will release a determined quantity of chemical species (that is equal on the average). The advantages of having a so optimized formulation guarantees effective release performances and provide the possibility of controlling(through illumination parameters such as light intensity and exposure time) the extent of the released chemical species.

Therefore, the density of gold nanorods preferably usable in a device formulated as described in Figure 2 corresponds to a gold concentration between 0.2 mM and 0.8 mM.

The effectiveness of this choice is confirmed by the matching (Figure 9) between the optical absorption spectrum of an aqueous solution containing gold nanorods and the spectrum of a release device as described above containing a gold concentration equal to 0.8 mM (that is below a threshold thereat plasmonic coupling takes place) as shown in figure 9 . Such matching indicates that the release device as described above and with a particle density below a certain threshold allows to keep the nanoparticles on average well separated one another.

In presence of a matrix with an optimum dispersion of nanoparticles, the quantity of released species can be modulated by varying fundamental illumination parameters such as the light power per surface unit and the exposure time to the light treatment. Figure 4 shows the absorption spectra of rhodamine 6G , which is released in physiological solution by porous matrixes of chitosan (3.5% w/v) doped with gold nanorods with an aspect ratio of 4 and a density of 0.8 mM of included gold, containing micelles of PCL-PEO-PCL (10 wt%) and 1% w/v of rhodamine 6G: on the left, upon varying the light intensity produced by an AIGaAs diode laser at 810 nm operating in continuous mode (cw) after 1 minute of illumination; on the right, upon varying the exposure time with a constant light intensity of 0.36 W/cm². The small squares show the values (in µg/mm³) of rhodamine 6G released in solution upon varying the parameters above. From the performed tests it results the possibility of controlling the released dose of chemical species by varying the radiation parameters, as it results from the substantially linear trend of the released quantity. It can be noticed that such trend can be progressively altered by the exhaustion of the species loaded inside the release device (not shown). The latter phenomenon can be controlled by acting upon the quantity of loaded chemical species, upon the density of structures of amphiphilic molecules in the device and upon the experimental conditions wherein the release takes place.

The temperature increase obtained as a consequence of the light stimulus can be controlled by varying the light intensity as shown in the diagram on the left of Figure 5 displaying the temperature profiles measured upon varying the light intensity (0.32 - 0.36 - 0.41 - 0.46W/cm²) produced by an AIGaAs diode laser at 810 nm operating in continuous mode (cw) for light stimuli of 1 minute on release devices constituted by matrixes of chitosan (3.5 % w/v) doped with gold nanorods with an aspect ratio equal to 4 and with a concentration of 0.8 mM, containing micelles of PCL-PEO-PCL (10 wt%). The linear relation between maximum temperature the and light intensity is evidenced in the right diagram of Figure 5.

The invention allows to obtain a light-induced release of molecules of a chemical species, by means of a porous polymeric matrix and a release device, having the following features:
- controllable in the released dosage depending upon light intensity and exposure time, at given concentrations of supramolecular structures of amphiphilic molecules and of loaded chemical species;
- repeatable in time as the effect of thermal activation of the supramolecular structures based upon amphiphilic molecules is reversible;
- implementable by means of a localized photothermal effect which can be controlled by the operator and kept under the level of irreversible heat damage to biological and tissue components adjacent to the device, by acting upon the light intensity and exposure time (that is upon the reached average temperature and upon the exposure time at that temperature);
- thermosensitive supramolecular structures , which are subjected to a modification in their average sizes and/or permeability or however in some chemical and/or structural property during illumination, which however does not influence the integrity, structure, mechanical properties and positioning of the porous matrix constituting the device;
- rapidly activatable, as the photothermal effect induces a temperature rise in short times, that is in the order of seconds.

### EXAMPLE

The example deals with the preparation, characterization and drug-release properties of a matrix constituted by chitosan structured in porous form and containing gold nanorods with aspect ratio of about 4, determining an optical absorption peak around 800 nm, including thermosensitive micelles of PCL-PEO-PCL with a thermal transition a little above the physiological temperature, (around 40°C), wherein said micelles are able to reduce the average sizes thereof by about one third beyond said thermal transition and, consequently, to stimulate the release of a previously-loaded chemical species which in the present case is represented by rhodamine 6G.

### Preparation

Low-molecular chitosan (average MW 120 kDa, Sigma-Aldrich) was dissolved to a final 3.5% (w/v) in an acidic dispersion of gold nanorods ((40 ± 6) nm x (10 ± 2) nm, 3.9± 0.5 aspect ratio, 0.8 mM of gold content). Fifty microliters of an ethanol solution of the copolymer (30 wt%) were rapidly injected into 150 µL of the nanorods-chitosan composite and mixed until complete homogenization. For the release measurements, the PCL-PEO-PCL alcoholic solution also included 1 mg mL⁻¹ of either rhodamine 6G , or Dox. Finally, 70 µL of the solution were poured into 0.5 cm² polystyrene circular moulds and after 180 min of evaporation of the solvent under controlled conditions (25 °C, N₂ flux), insolubilization of the matrix was obtained by treatment with 1 M NaOH followed by abundant rinsing with ultrapure water. Samples were stored in ultrapure water at 4°C until further investigation.

### Characterization

The in-solution release from the release device was evaluated in PBS (pH 7.4) at different temperature values (produced by a Thermo Blok 780, Asal). The buffer solution surrounding the device was collected and replaced with fresh buffer after each period of treatment. The cumulative percentage of released rhodamine 6G is calculated by the maximum absorption values measured at the peak of rhodamine 6G at 530 nm. Figure 6 shows an enhanced chemical release from the device when it is subjected to a temperature increase above the physiological value, which differently is not observed at lower values.

Different combinations of laser intensities and irradiation times were performed as illustrated in Figure 7. At the beginning and at the end of each illumination period the optical absorption spectrum of the solution was measured and replaced with fresh buffer.

Figure 7 shows that a repeated and customized release of rhodamine 6G from the device is feasible, which underlines the system reversibility and inspires the concept of customized pharmacological therapies.

The above-described device keeps unaltered its initial size after light illumination as underlined by the evaluation of the deswelling ratio (DSR). Such ratio was obtained by measuring the weight of the device before undergoing laser illumination (t₀) and after 1 minute of illumination (t₁) and calculated according to the expression: DSR = 100 x ((Weight(t₁)-Weight(t₀))/Weight(t₀)); the result is a decrease in weight of ∼ 1% for time durations up to 30 minutes of light treatment performed with an AIGaAs diode laser at 810 nm operated in continuos wave (cw) mode and a light intensity of 0.41 W cm⁻², which can be associated to a negligible variation in the initial volume and however within the limits of the measurement error. This feature is the result of the presence of a bearing structure (polymer matrix) which is not subjected to structural modifications in the thermal interval of interest for the release and which, viceversa, includes micelles which have a thermal transition in the involved interval, as shown in Figure 8, which determines a consistent contraction of the volume thereof, as shown in Figure 9, but which does not influence the whole matrix structure.

More in detail, the comparison between the thermograms of a device without micelles of PCL-PEO-PCL and a device containing micelles of PCL-PEO-PCL (see Figure 8), underlines the absence of important variations in the temperature range of 15 ÷ 65°C in the first case and, viceversa, an endothermal transition with peak at about 40°C in the second case. This transition can then be ascribed to the presence of the PCL-PEO-PCL micelles included in the device. The DLS analysis (Figure 9) underlines that PCL-PEO-PCL micelles undergo a micellar contraction of about one third of the volume thereof upon varying the temperature from lower values (25 °C) to higher values (50 °C) of the transition temperature.

### Drug release in cell cultures

HeLa cervical cancer cells (ATCC) were cultured in phenol-red-free DMEM supplemented with 10% fetal bovine serum (FBS), 1 mM glutamine, 100 U mL⁻¹ penicillin and 100 µg mL⁻¹ streptomycin in 5% CO₂ at 37°C. Laser experiments were performed using a bench setup consisting of an AIGaAs diode laser peaked at 810 nm coupled with a 600 µm core optical fiber and a precision stage with a few mm sized hole..

Matrices loaded with Dox were placed in contact with a HeLa cell monolayer. Various irradiation conditions induced different yields of cell death, as was revealed by the calcein (CA)/propidium iodide (PI) assay after 4 h from laser treatment (Figure 10). As expected, larger exposure times and laser intensities were shown to provide more effective chemotherapy, in accordance with the copious PI staining at the expense of CA-positive viable cells. These results are plotted as a function of internalized Dox, expressed as intracellular Dox fuorescence of cell samples treated under different laser conditions. The low supraphysiological temperatures (40-45 °C, see Figure 5) reached upon laser-activation of the device by using the lighy intensities of the experiments are considered ineffective in inducing any irreversible thermal damages to cells. This circumscribes the cytotoxic effects observed to the antitumor Dox activity. The latter is enhanced by the synergic assistance of a hyperthermic effect, which stimulates drug uptake by temporarily increasing the plasmatic membrane permeability confirmed by the prevalence of PI-stained cells adjacent to the irradiated portion of the sponge, as can be observed under the optimal conditions 0.46 W cm⁻² and tᵢᵣᵣ= 5 min (figure 11)).

## Claims

1. Porous polymeric matrix transparent to a light flux in the visible or NIR spectrum comprising: metallic nanometric particles absorbing light in the visible or in the NIR spectrum and thermosensitive micellar structures constituted by amphiphilic block copolymers containing chemical species, wherein said metallic nanometric particles have a substantially homogeneous distribution within said porous polymeric matrix and said micellar structures constituted by block copolymers are dispersed in and constrained to said porous polymeric matrix.

2. Porous polymeric matrix of claim 1 for use for the delivery of chemicals.

3. Porous polymeric matrix of claim 2 for use for the delivery of anticancer agents.

4. A device for optically-controlled release of chemical species, comprising:
• a porous polymeric matrix with pores with size so as to allow the passage of a chemical species to be delivered, said matrix being substantially transparent to a light flux;
• a plurality of metallic nanometric particles dispersed in said porous polymeric matrix with a substantially homogeneous distribution, apt to be excited when they are invested by said light flux by producing heat; and
• a plurality of thermosensitive micellar structures constituted by amphiphilic block copolymers, including said chemical species to be delivered at a predetermined administration temperature, said micellar structures constituted by amphiphilic block copolymers being dispersed and constrained to said porous polymeric matrix,
wherein said metallic nanometric particles and said micellar structures constituted by amphiphilic block copolymers are distinct to each other following to different dispersions
the metallic nanometric particles being apt to increase the average temperature of the porous polymeric matrix at said predetermined administration temperature when the porous polymeric matrix is illuminated by a light flux at a predetermined illumination intensity, the metallic nanometric particle dispersion being chosen to not affect the structural integrity of the porous polymeric matrix at said predetermined administration temperature.

5. The device according to claim 4, wherein the porous polymeric matrix is provided in the shape of a thin film with a thickness comprised between 10 µm and 1000 µm and preferably between 40 µm and 500 µm.

6. The release device according to claim 4, wherein the pores of the porous polymeric matrix have sizes comprised in the range of 10 nm ÷ 5000 nm, preferably between 50 and 500 nm.

7. The release device according to claim 4, wherein said chemical species comprises at least a pharmacological agent, in particular an antitumour pharmacological agent.

8. The release device according to claim 4, wherein said metallic nanometric particles are apt to be excited at determined frequencies of plasmonic resonance if illuminated by a light beam and are gold nanorods.

9. The release device according to claim 8, wherein the metallic nanometric particles have sizes and an aspect ratio so as to be excited at determined frequencies of plasmonic resonance when illuminated by a light flux with wavelength comprised in the range of 500 ÷ 1200 nm.

10. The release device according to claim 8, wherein said metallic nanometric particles have preferential sizes between 20 nm and 120 nm of length and 5 nm and 30 nm of diameter.

11. The release device according to claim 8, wherein said metallic nanometric particles inside said porous polymeric matrix under hydrated form are comprised in the range of 0.0001 ÷ 1 wt% and preferably 0.001 ÷ 0.1 wt%.

12. The release device according to claim 8, wherein said porous polymeric matrix is a hydrogel.

13. The release device according to claim 12, wherein said hydrogel comprises chitosan.

14. The release device according to claim 13, wherein the porous polymeric matrix made of chitosan is prepared for deposition of an aqueous solution at acid pH of chitosan with a subsequent solvent evaporation performed in the temperature range of 20 ÷ 35 °C.

15. The release device according to claim 14, wherein said evaporation is terminated by producing matrix insolubilization by means of alkalinisation for a period preferably comprised between 1 and 30 minutes, followed by one or more passages of neutralization in water, before the complete evaporation of the solvent takes place, by leaving to pass a time comprised between 30 minutes and 6 hours from the deposition.

16. The release device according to claim 4, wherein said micellar structures constituted by block copolymers includes chains of polycaprolactone (PCL) and polyethylene oxide (PEO).

17. The release device according to claim 8, wherein the gold concentration under the form of nanorods is preferably comprised in the range between 0.2 mM and 0.8 mM.

18. The release device according to claim 8, wherein the minimum distance between gold nanorods is equal or higher than 2.5 times the diameter of the metallic nanometric particle.

19. The release device according to claim 4, wherein said metallic nanometric particles are coated by an organic material, preferably polyethylene glycol (PEG) or inorganic material, preferably silica, or titania.

## Patentansprüche

1. Poröse polymere Matrix, transparent für einen Lichtfluss im sichtbaren oder NIR-Spektrum, umfassend: metallische nanometrische Partikel, die Licht im sichtbaren oder im NIR-Spektrum absorbieren und wärmeempfindliche mizellare Strukturen, gebildet aus amphiphilen Blockcopolymeren, welche chemische Spezies enthalten, wobei die genannten metallischen nanometrischen Partikel eine im Wesentlichen homogene Verteilung innerhalb der genannten porösen polymeren Matrix haben und die durch Blockcopolymere gebildeten genannten mizellaren Strukturen in der genannten porösen polymeren Matrix dispergiert und eingelagert sind.

2. Poröse polymere Matrix, nach Anspruch 1, für die Verwendung zur Freisetzung von Chemikalien.

3. Poröse polymere Matrix, nach Anspruch 2, für die Verwendung zur Freisetzung von Antikrebswirkstoffen.

4. Vorrichtung zur optisch kontrollierten Freisetzung von chemischen Spezies, umfassend:
• eine poröse polymere Matrix mit Poren in einer Größe, um einer freizusetzenden chemischen Spezies den Durchgang zu ermöglichen, wobei die genannte Matrix im Wesentlichen transparent für einen Lichtfluss ist;
• eine Vielzahl von metallischen nanometrischen Partikeln, dispergiert mit einer im Wesentlichen homogenen Verteilung in der genannten porösen polymeren Matrix, geeignet angeregt zu werden, wenn die durch den genannten Lichtfluss erzeugte Wärme angelegt wird; und
• eine Vielzahl von wärmeempfindlichen mizellaren Strukturen, gebildet aus amphiphilen Blockcopolymeren, enthaltend die genannten chemischen Spezies, die bei einer vorherbestimmten Verabreichungstemperatur abgegeben werden, wobei die durch amphiphile Blockcopolymere gebildeten genannten mizellaren Strukturen in der genannten porösen polymeren Matrix dispergiert und eingelagert sind,
worin die genannten metallischen nanometrischen Partikel und die durch amphiphile Blockcopolymere gebildeten genannten mizellaren Strukturen sich voneinander durch die folgenden verschiedenen Dispersionen unterscheiden
die metallischen nanometrischen Partikel sind geeignet, die mittlere Temperatur der porösen polymeren Matrix bei der genannten vorherbestimmten Verabreichungstemperatur zu erhöhen, wenn die poröse polymere Matrix durch einen Lichtfluss bei einer vorherbestimmten Beleuchtungsstärke beleuchtet wird, die Dispersion der metallischen nanometrischen Partikel derart gewählt ist, dass die strukturelle Integrität der porösen polymeren Matrix bei der genannten vorherbestimmten Verabreichungstemperatur nicht beeinträchtigt wird.

5. Vorrichtung, gemäß Anspruch 4, worin die poröse polymere Matrix in Form eines dünnen Films vorgesehen ist, mit einer Dicke umfassend zwischen 10 µm und 100 µm und vorzugsweise zwischen 40 µm und 500 µm.

6. Freisetzungsvorrichtung, gemäß Anspruch 4, worin die Poren der porösen polymeren Matrix Größen haben, umfassend den Bereich von 10 nm ÷ 5000 nm, vorzugsweise zwischen 50 und 500 nm.

7. Freisetzungsvorrichtung, gemäß Anspruch 4, worin die genannten chemischen Spezies wenigstens einen pharmakologischen Wirkstoff umfassen, insbesondere einen antitumoralen pharmakologischen Wirkstoff.

8. Freisetzungsvorrichtung, gemäß Anspruch 4, worin die genannten metallischen nanometrischen Partikel geeignet sind, um bei vorherbestimmten Frequenzen der plasmonischen Resonanz angeregt zu werden, wenn diese durch einen Lichtstrahl beleuchtet werden und Goldnanostäbchen sind.

9. Freisetzungsvorrichtung, gemäß Anspruch 8, worin die metallischen nanometrischen Partikel Größen und ein Aspektverhältnis aufweisen, um bei vorherbestimmten Frequenzen der plasmonischen Resonanz angeregt zu werden, wenn diese durch einen Lichtfluss mit einer Wellenlänge umfassend den Bereich von 500 ÷ 1200 nm beleuchtet werden.

10. Freisetzungsvorrichtung, gemäß Anspruch 8, worin die genannten metallischen nanometrischen Partikel bevorzugte Größen zwischen 20 nm und 120 nm in der Länge und 5 nm und 30 nm im Durchmesser haben.

11. Freisetzungsvorrichtung, gemäß Anspruch 8, worin die genannten metallischen nanometrischen Partikel innerhalb der genannten porösen polymeren Matrix unter hydratisierter Form in einem Bereich von 0,0001 ÷ 1 Gew.-% und vorzugsweise 0,001 ÷ 0,1 Gew.-% enthalten sind.

12. Freisetzungsvorrichtung, gemäß Anspruch 8, worin die genannte poröse polymere Matrix ein Hydrogel ist.

13. Freisetzungsvorrichtung, gemäß Anspruch 12, worin das Hydrogel Chitosan umfasst.

14. Freisetzungsvorrichtung, gemäß Anspruch 13, worin die aus Chitosan gebildete poröse polymere Matrix hergestellt ist durch Ausfällung von Chitosan aus einer wässrigen Lösung mit einem sauren pH-Wert, mit einer in einem Temperaturbereich von 20 ÷ 35 °C durchgeführten nachfolgenden Lösemittelverdampfung.

15. Freisetzungsvorrichtung, gemäß Anspruch 14, worin die genannte Verdampfung durch Erzeugung einer Unlöslichkeit der Matrix mittels Alkalisierung in einem Zeitraum umfassend zwischen 1 und 30 Minuten beendet wird, gefolgt von einem oder mehreren Arbeitsgängen der Neutralisation mit Wasser, bevor die vollständige Verdampfung des Lösemittels stattfindet, mit einem Zeitablauf nach der Ausfällung umfassend zwischen 30 Minuten und 6 Stunden.

16. Freisetzungsvorrichtung, gemäß Anspruch 4, worin die durch Blockcopolymere gebildeten genannten mizellaren Strukturen Ketten von Polycaprolacton (PCL) und Polyethylenoxid (PEO) enthalten.

17. Freisetzungsvorrichtung, gemäß Anspruch 8, worin die Goldkonzentration in Form von Nanostäbchen vorzugsweise im Bereich zwischen 0,2 mM und 0,8 mM umfasst ist.

18. Freisetzungsvorrichtung, gemäß Anspruch 8, worin der Mindestabstand zwischen Goldnanostäbchen gleich oder größer als der 2,5-fache Durchmesser der metallischen nanometrischen Partikel ist.

19. Freisetzungsvorrichtung, gemäß Anspruch 4, worin die genannten metallischen nanometrischen Partikel mit einem organischen Material, vorzugsweise Polyethylenglycol (PEG), oder anorganischem Material, vorzugsweise Siliziumdioxid oder Titandioxid, beschichtet sind.

## Revendications

1. Matrice polymère poreuse transparente à un flux lumineux dans le spectre visible ou proche infrarouge comprenant : des particules métalliques nanométriques absorbant la lumière dans le spectre visible ou proche infrarouge et des structures micellaires thermosensibles constituées de copolymères séquencés amphiphiles contenant une espèce chimique, dans laquelle lesdites particules métalliques nanométriques présentent une répartition sensiblement homogène au sein de ladite matrice polymère poreuse et lesdites structures micellaires constituées de copolymères séquencés sont dispersées et contraintes dans ladite matrice polymère poreuse.

2. Matrice polymère poreuse selon la revendication 1 pour son utilisation pour délivrer des produits chimiques.

3. Matrice polymère poreuse selon la revendication 2 pour son utilisation pour délivrer des agents anticancéreux.

4. Dispositif pour la libération optiquement contrôlée d'espèces chimiques, comprenant :
• une matrice polymère poreuse ayant des pores ayant une taille permettant le passage d'une espèce chimique à délivrer, ladite matrice étant sensiblement transparente à un flux lumineux ;
• une pluralité de particules métalliques nanométriques dispersées dans ladite matrice polymère poreuse avec une répartition sensiblement homogène, aptes à être excitées quand elles sont investies par ledit flux lumineux en produisant de la chaleur ; et
• une pluralité de structures micellaires thermosensibles constituées de copolymères séquencés amphiphiles, incluant ladite espèce chimique à délivrer à une température d'administration prédéterminée, lesdites structures micellaires constituées de copolymères séquencés amphiphiles étant dispersées et contraintes dans ladite matrice polymère poreuse,
dans lequel lesdites particules métalliques nanométriques et lesdites structures micellaires constituées de copolymères séquencés amphiphiles sont distinctes les unes des autres après différentes dispersions
les particules métalliques nanométriques étant aptes à augmenter la température moyenne de la matrice polymère poreuse à ladite température d'administration prédéterminée quand la matrice polymère poreuse est éclairée par un flux lumineux à une intensité d'éclairage prédéterminée, la dispersion des particules métalliques nanométriques étant choisie de manière à ne pas affecter l'intégrité structurelle de la matrice polymère poreuse à ladite température d'administration prédéterminée.

5. Dispositif selon la revendication 4, dans lequel la matrice polymère poreuse est fournie sous la forme d'un film mince ayant une épaisseur comprise entre 10 µm et 1000 µm et de préférence entre 40 µm et 500 µm.

6. Dispositif de libération selon la revendication 4, dans lequel les pores de la matrice polymère poreuse présentent des tailles comprises dans la plage allant de 10 nm à 5000 nm, de préférence entre 50 et 500 nm.

7. Dispositif de libération selon la revendication 4, dans lequel ladite espèce chimique comprend au moins un agent pharmacologique, en particulier un agent pharmacologique anti-tumeur.

8. Dispositif de libération selon la revendication 4, dans lequel lesdites particules métalliques nanométriques sont aptes à être excitées à des fréquences déterminées de résonance plasmonique si elles sont éclairées par un faisceau lumineux et sont des nanobâtonnets en or.

9. Dispositif de libération selon la revendication 8, dans lequel les particules métalliques nanométriques présentent des tailles et un rapport d'aspect afin d'être excitées à des fréquences déterminées de résonance plasmonique quand elles sont éclairées par un flux lumineux ayant une longueur d'onde comprise dans la plage allant de 500 à 1200 nm.

10. Dispositif de libération selon la revendication 8, dans lequel lesdites particules métalliques nanométriques présentent des tailles préférentielles entre 20 nm et 120 nm de longueur et 5 nm et 30 nm de diamètre.

11. Dispositif de libération selon la revendication 8, dans lequel lesdites particules métalliques nanométriques à l'intérieur de ladite matrice polymère poreuse sous forme hydratée sont comprises dans la plage allant de 0,0001 à 1 % en poids et de préférence de 0,001 à 0,1 % en poids.

12. Dispositif de libération selon la revendication 8, dans lequel ladite matrice polymère poreuse est un hydrogel.

13. Dispositif de libération selon la revendication 12, dans lequel ledit hydrogel comprend du chitosane.

14. Dispositif de libération selon la revendication 13, dans lequel la matrice polymère poreuse faite de chitosane est préparée pour un dépôt d'une solution aqueuse à un pH acide de chitosane avec une évaporation ultérieure de solvant réalisée dans la plage de températures allant de 20 à 35 °C.

15. Dispositif de libération selon la revendication 14, dans lequel ladite évaporation est terminée en produisant une insolubilisation de matrice au moyen d'une alcalinisation pendant une période comprise de préférence entre 1 et 30 minutes, suivie d'un ou de plusieurs passages de neutralisation dans l'eau, avant que l'évaporation complète du solvant ait lieu, en laissant passer un temps compris entre 30 minutes et 6 heures à partir du dépôt.

16. Dispositif de libération selon la revendication 4, dans lequel lesdites structures micellaires constituées de copolymères séquencés comprennent des chaînes de polycaprolactone (PCL) et de polyoxyde d'éthylène (PEO).

17. Dispositif de libération selon la revendication 8, dans lequel la concentration en or sous forme de nanobâtonnets est comprise de préférence dans la plage allant de 0,2 mM à 0,8 mM.

18. Dispositif de libération selon la revendication 8, dans lequel la distance minimale entre les nanobâtonnets en or est supérieure ou égale à 2,5 fois le diamètre de la particule métallique nanométrique.

19. Dispositif de libération selon la revendication 4, dans lequel lesdites particules métalliques nanométriques sont revêtues d'un matériau organique, de préférence un polyéthylène glycol (PEG) ou d'un matériau inorganique, de préférence la silice, ou l'oxyde de titane.
